# EUROPEAN PATENT APPLICATION

(11) **EP 3 960 066 A1**
(43) Date of publication of application: **02.03.2022**
(21) Application number: 20192711.8
(22) Date of filing: 25.08.2020
(51) Int. Cl.: A61B 3/10, A61B 3/13, A61B 3/15, G01B 11/24, G02B 7/00, G02B 21/00, G01B 9/02

(54) **CONTROL SYSTEM FOR AN OCT IMAGING SYSTEM, ARRANGEMENT WITH AN OCT IMAGING SYSTEM AND METHOD FOR ADJUSTING AN OCT IMAGING SYSTEM**

(71) Applicant: Leica Microsystems Inc., Buffalo Grove IL 60089 (US)
(72) Inventor: HART, Robert H., Cary, NC 27511 (US); BRANCO, Dorothy M., Durham, NC 27713 (US); LYNCH, Eric, Apex, NC 27523 (US)
(74) Representative: DehnsGermany Partnerschaft von Patentanwälten

(57) **Abstract**

The invention relates to a control system for an optical coherence tomography imaging system to be used with a microscopy system for viewing and/or imaging a subject (190, 192), the microscopy system comprising an objective (164) and a viewing lens system (170) including a relay lens (172), wherein the viewing lens system (170) is arranged at a subject's side of the objective (164), the control system being configured to perform the following steps: controlling the optical coherence tomography imaging system to perform at least one radial scan of a surface (176) of the relay lens (172), determining, from data of the at least one radial scan at least one curve corresponding to a shape of the surface (176) of the relay lens (172), determining (520), from the at least one curve a lateral offset (178) between a center of the relay lens (172) and an origin of the optical coherence tomography imaging system, and adjusting the origin of the optical coherence tomography imaging system taking into account to the lateral offset (178), to an arrangement with an OCT imaging system and a method for adjusting an OCT imaging system.

## Description

### Technical Field

The present invention essentially relates to a control system for an optical coherence tomography (OCT) imaging system to be used with a microscopy system for viewing and/or imaging a subject, to an arrangement with an OCT imaging system including such control system, and to a method for adjusting such an OCT imaging system.

### Background

Optical coherence tomography (in the following also called OCT, its typical abbreviation) is an imaging technique that uses low-coherence light to capture two- and three-dimensional images from within optical scattering media (e.g., biological tissue) with high resolution. It is, inter alia, used for medical imaging. Optical coherence tomography is based on low-coherence interferometry, typically employing near-infrared light The use of relatively long wavelength light allows it to penetrate into the scattering medium.

A medical field of particular interest for OCT is ophthalmology, a branch of medicine related to (in particular human) eyes and its disorders and related surgeries. In ophthalmology, an OCT imaging system typically is used in combination with a microscope, in particular an ophthalmic surgical microscope, or a corresponding microscopy system. A retinal or fundus viewing lens system (in the following also called RVLS) can be used as an accessory to such an ophthalmic surgical microscope in ophthalmic surgery to view the posterior segment (retina) of the eye. The RVLS has to be properly arranged with respect to the objective lens in order to minimize or prevent any misalignment.

### Summary

According to the invention, a control system, an arrangement with an OCT imaging system and a method for adjusting an OCT imaging system with the features of the independent claims are proposed. Advantageous further developments form the subject matter of the dependent claims and of the subsequent description.

The present invention relates to a control system for an optical coherence tomography (OCT) imaging system to be used with a microscopy system for viewing and/or imaging a subject The subject, preferably, includes or is an eye, in particular, a retina of the eye. The microscopy system comprises an objective or objective lens (which is part of the microscope) and a viewing lens system including a relay lens and, preferably, a relay lens. The viewing lens system is arranged at a subject's side of the objective and, preferably, is an attachable (and removable) accessory to the microscope. Thus, the viewing lens system, preferably, is or includes a retinal viewing lens system (RVLS). The microscopy system can include a (ophthalmic) surgical microscope.

The RVLS typically makes no contact with the eye and is intended for use with compatible ophthalmic microscopes and objective lenses. The reduction lens of the RVLS principally is used to reduce the effective focal length of the objective lens, and the relay lens transfers the focal plane of the microscope objective lens to the retina. An appropriate reduction lens should be selected for compatibility with the microscope's objective lens focal length. The focal length of the relay lens should be selected for the desired retinal image field of view. The position of the relay lens is adjustable to accommodate the refractive error of the patient eye thus providing a properly focused image on the subject retina.

The combination of reduction lens plus relay lens making up the RVLS can easily be inserted into the optical path of the (ophthalmic surgical) microscope. Care must be taken to properly adjust the microscope to ensure the subject eye is at the proper working distance (i.e. focal plane), of the objective lens before the RVLS is inserted in the optical path. If this condition is met, the RVLS can then be placed in the optical path without the need to further adjust the position of the microscope.

A proper microscope setup allows for all viewing modalities such as camera view, ocular view, and OCT view to be at the proper focus for both conditions where the RVLS is in use or out of use by removal from the optical path of the microscope.

It has been recognized that, due to the nature of a mechanical interface between the RVLS and the microscope, an offset of the RVLS relative to the optical axis of the objective lens in either a lateral displacement or angular tilt can exist. In such cases, an OCT scan typically is not centered on the optical axis of the eye, thus, manual adjustment of the OCT scan origin relative to the optical axis of the subject eye during the surgical procedure is required. Pre-set alignment offsets typically are ineffectual since they are established based on either a theoretical model or good condition RVLS, i.e., one in perfect alignment. If the RVLS is not properly attached or is aged and no longer in perfect alignment, preset alignment values will not properly center the OCT beam to the optical axis of the eye.

The manual adjustment to re-center the OCT scan origin to the optical axis of the eye typically requires multiple iterations which can be a frustrating process for the surgeon by adding an extra challenge to the surgeon in having to think about the scan origin offset magnitude and how it relates to the anatomy of the eye. The process is somewhat subjective which can lead to uncertainty by the surgeon as to the exact location of the OCT scan relative to the anatomy of the subject eye in addition to extending the length of the surgical procedure.

In view of these drawbacks, the control system within an embodiment of the present invention is configured to control the OCT imaging system to perform at least one radial scan of a surface of the relay lens. Preferably, it is configured to perform at least two or even more different radial scans. Further, it is configured to determine, from (scan) data of the at least one radial scan, at least one curve corresponding to a shape of the surface of the relay lens. In particular, it is also configured to determine at least two different curves corresponding to the shape of the surface of the relay lens (their number corresponding to the number of radial scans).

Further, the control system is configured to determine, from the at least one curve, a lateral offset between a center of the relay lens and an origin of the OCT imaging system. In particular, this determining, from the at least one curve, the lateral offset comprises: determining an apex (or highest or peak point) of the at least one curve (or of each of several curves), determining a lateral position of the apex (which typically includes x- and y-coordinates), and determining the lateral offset from a difference of the lateral position of the apex and the origin of the optical coherence tomography imaging system. Further, the control system is configured to adjust the origin of the OCT imaging system taking into account to the lateral offset

In addition, the control system can be configured to determine, from the at least one curve, an angular offset between a center axis of the relay lens and an optical axis of the microscopy system, and to adjust the OCT imaging system taking into account to the angular offset

These technical features aim to automate OCT scan centration on the RVLS and correct any lateral or angular offsets between the optical axis of the microscope and the RVLS. With the RVLS in place and focused on the retina, the control system will allow the surgeon to center the OCT scan origin on the RVLS with a single command. The lateral (x and y) offsets and any angular offsets (tilt) will be automatically calculated and set in software accordingly.

To automatically correct for the mechanical lateral offset or tilt the OCT scan refers to the microscope image or video, an OCT scan can be obtained of the top surface of the relay lens. The shape of the relay lens on the OCT scan will resemble a convex or dome shaped curve. From this surface curvature, the lateral (i.e. x and y) position the apex of the curvature can be determined. The calculated offsets can then be automatically applied to the OCT scan origin, thus, centering the OCT scan to the optical axis of the RVLS and hence the subject eye as well. Verification of accurate centering is, for example, apparent when the so-called surface Purkinje reflection (Purkinje images are reflections of objects from the structure of the eye) is coincident with the OCT scan origin.

The embodiments of the invention can provide several advantages, in particular in intra-surgical OCT space. The improved location accuracy will allow such control system to be considered for applications where accuracy of location of visualization is needed, including sub-retinal injections and assessing relative position of anatomical features. Confidence of the Surgeon in the actual location of the OCT scan can be increased, which means higher trust in the information that is being visualized.

Workflow time is reduced as the system automatically adjusts for any lateral or angular mechanical offset, thus, eliminating the need to both determine and adjust the OCT scan during surgery. Pre-operative system setup time is reduced by eliminating manual recentering of the OCT scan origin for each RVLS. Also, automatic centration removes any residual errors that would be introduced during standard RVLS use (mounting, unmounting, cleaning cycles). These types of errors cannot be compensated for by the use of pre-loaded scan alignment offsets.

The invention also relates to an arrangement comprising an OCT imaging system and a microscopy system for viewing and/or imaging a subject (like a retina of an eye), the microscopy system comprising an objective and a viewing lens system including a relay lens, wherein the viewing lens system is arranged at the subject's side of the objective, further comprising a control system according to the invention.

Further, the invention also relates to a method for adjusting an OCT imaging system to be used with a microscopy system for viewing and/or imaging a subject, the microscopy system comprising an objective and a viewing lens system including a relay lens, wherein the viewing lens system is arranged at a subject's side of the objective (i.e., between the objective and the subject). The method comprises the following steps: performing at least one radial scan of a surface of the relay lens, by means of the optical coherence tomography imaging system; determining, from data of the at least one radial scan, at least one curve corresponding to a shape of the surface of the relay lens; determining, from the at least one curve, a lateral offset between a center of the relay lens and an origin of the optical coherence tomography imaging system; and adjusting the origin of the optical coherence tomography imaging system taking into account to the lateral offset. Preferably, the viewing lens system is attached to the objective before performing the at least one radial scan.

With respect to further preferred embodiments or details and advantages of the arrangement and the method, it is also referred to the remarks for the control system above, which apply here correspondingly.

The invention also relates to a computer program with a program code for performing a method according to the invention when the computer program is run on a processor or on a control system according to the invention.

Further advantages and embodiments of the invention will become apparent from the description and the appended figures.

It should be noted that the previously mentioned features and the features to be further described in the following are usable not only in the respectively indicated combination, but also in further combinations or taken alone, without departing from the scope of the present invention.

Short Description of the Figures
- Fig. 1: shows a schematic overview of an arrangement with an OCT imaging system according to the invention in a preferred embodiment
- Fig. 2: shows, schematically, part of a microscopy system as used within an embodiment of the invention.
- Fig. 3: shows, schematically, a ray-trace diagram with laterally misaligned RVLS.
- Fig. 4: shows, schematically, a ray-trace diagram with angularly misaligned RVLS.
- Fig. 5: shows, schematically, a flow scheme describing a method according to the invention in a preferred embodiment
- Fig. 6: shows, schematically, OCT images for explanation of an aspect of the invention.
- Fig. 7: shows, schematically, a ray-trace diagram with laterally misaligned RVLS, and the OCT imaging system origin being corrected.
- Fig. 8: shows, schematically, a ray-trace diagram with angularly misaligned RVLS, and the OCT imaging system origin being corrected.

### Detailed Description

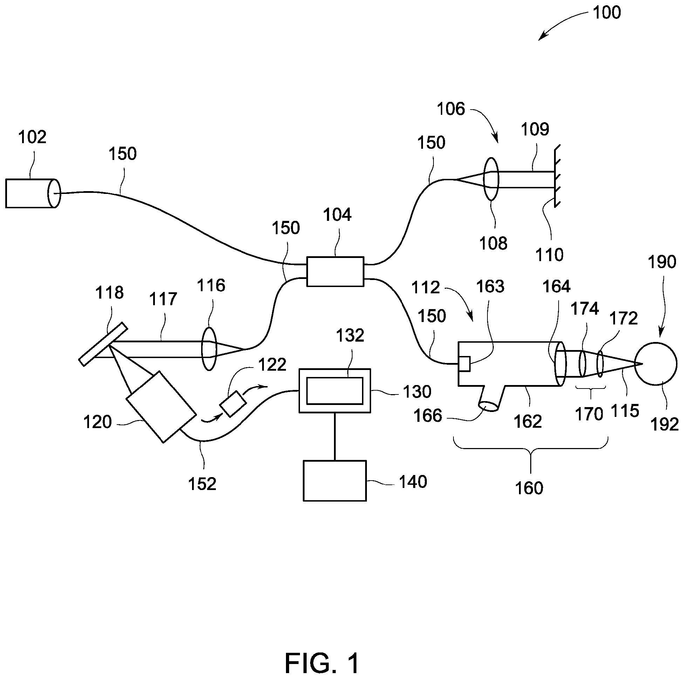

In Fig. 1, a schematic overview of an arrangement with an optical coherence tomography (OCT) imaging system 100 and a microscopy system 160, in a preferred embodiment, is shown. The OCT imaging system 100 comprises a light source 102 (e.g., a low coherence light source), a beam splitter 104, a reference arm 106, a sample arm 112, a diffraction grating 118, a detector 120 (e.g., a camera), a control system 130 and display means 140 (e.g., a display or monitor).

Light originating from the light source 102 is guided, e.g., via fiber optic cables 150, to the beam splitter 104 and a first part of the light is transmitted through the beam splitter 104 and is then guided, via a lens 108 (which is only schematically shown and may represent also different, appropriate optics) in order to create a light beam 109 to a reference mirror 110, wherein the lens 108 and the reference mirror 110 are part of the reference arm 106.

Light reflected from the reference mirror 110 is guided back to the beam splitter 104 and is transmitted through the beam splitter 104 and is then guided, via a lens 116 (which is only schematically shown and may represent also different, appropriate optics) in order to create a light beam 117 to the diffraction grating 118.

A second part of the light, originating from the light source 102 and transmitted through the beam splitter 104 is guided, via microscopy system 160 (which is only schematically shown) in order to create a light beam 115 (for scanning) to the subject or subject eye 190 to be imaged and which comprises a retina 192.

The microscopy system 160 is part of the sample arm 112 and comprises a microscope 162 (e.g., an ophthalmic surgical microscope) and a retinal viewing lens system (RVLS) 170, which in turn comprises a relay lens 172 and a reduction lens 174. The microscope 162 includes a light input and output 163 (for the light originating from the light source 102), an ocular 166 and an objective or objective lens 164 (might also include a plurality of lenses).

Light reflected from the subject eye 190 or the retina 192 is guided back to the beam splitter 104 and is transmitted through the beam splitter 104 and is then guided, via lens 116 to the diffraction grating 118. Thus, light reflected in the reference arm 106 and light reflected in the sample arm 112 are combined by means of the beam splitter 104 and are guided, e.g., via a fiber optic cable 150, and in a combined light beam 117 to the diffraction grating 118.

Light reaching the diffraction grating 118 is diffracted and captured by the detector 120. In this way, the detector 120, which acts as a spectrometer, creates or acquires data or scan data 122 that are transmitted, e.g., via an electrical cable 152, to the control system 130 comprising processing means (or a processor) 132. The scan data 122 can then processed to obtain image data that are transmitted, e.g., via an electrical cable, to the display means 140 and displayed as a real-time image, i.e., an image that represents the currently scanned subject 190 in real-time. The process in which the scan data 122 is processed to determine the lateral and/or angular offset will be described in more detail in the following.

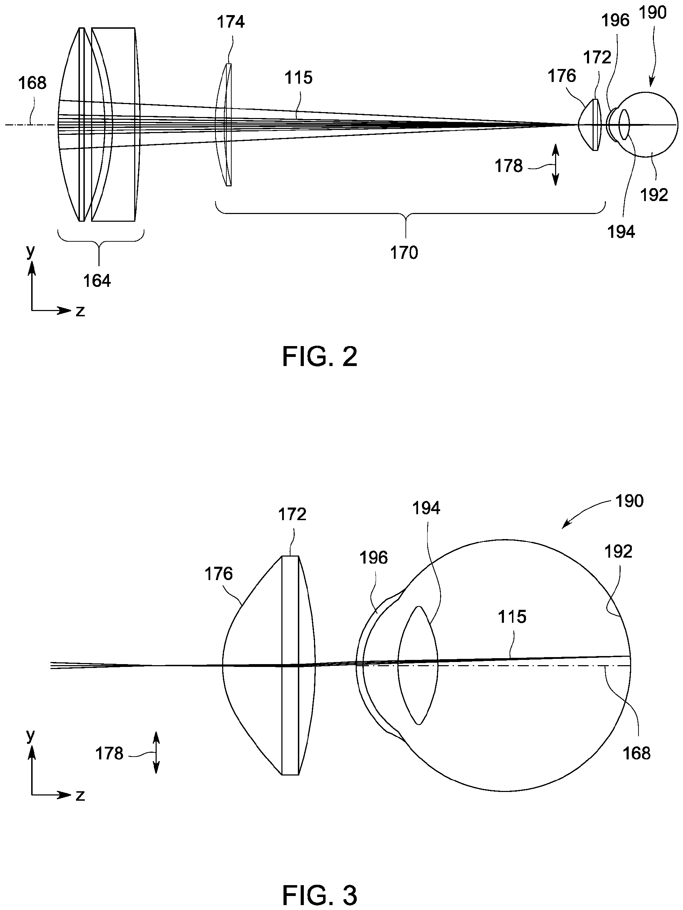

In Fig. 2, part of the microscopy system 160 as used within the invention and as indicated in Fig. 1, is shown in more detail. On the left side, the objective of objective lens 164 of the microscope, defining an optical axis 168 (which is also the optical axis of the OCT imaging system), is shown. Light emerging from the objective 164 passes the reduction lens 174 and the relay lens 172 (including its surface 176), enters the eye 190 through the cornea 196, passes the eye lens 194 and, reaches the retina 192.

In the situation shown, the RVLS 170, comprising the reduction lens 174 and the relay lens 172, is aligned with the optical axis 168 and, thus, does not have a lateral offset The lateral offset, in general, is denoted by reference numeral 178, and includes, e.g., a y-component. Imaging coordinates of the OCT imaging system comprise x-, y- and z-coordinates (only y- and z-coordinates shown in Fig. 2).

In Fig. 3, a ray-trace diagram with laterally misaligned RVLS is shown. The basic system corresponds to the one shown in Fig. 2. However, the RVLS (including the relay lens 172) is laterally misaligned, i.e., there is a lateral offset 178. This results in the OCT beam (which the origin of the OCT imaging system being at its default position) deviating from the optical axis, as can be seen.

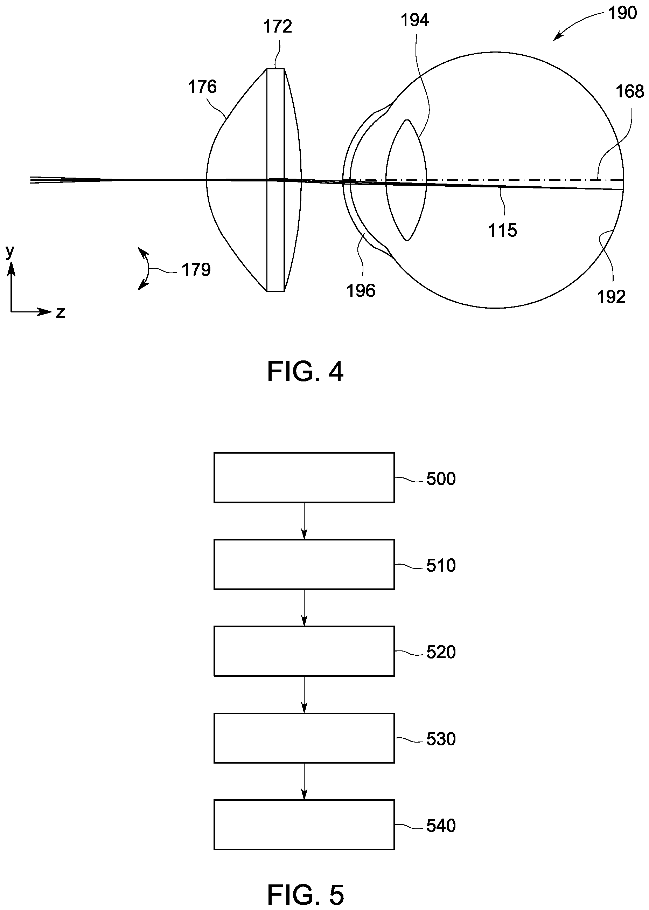

In Fig. 4, a ray-trace diagram with angularly misaligned RVLS is shown. The basic system corresponds to the one shown in Figs. 2 and 3. However, the RVLS (including the relay lens 172) is angularly misaligned or tilted, i.e., there is an angular offset 179. This results in the OCT beam (which the origin of the OCT imaging system being at its default position) deviating from the optical axis, as can be seen.

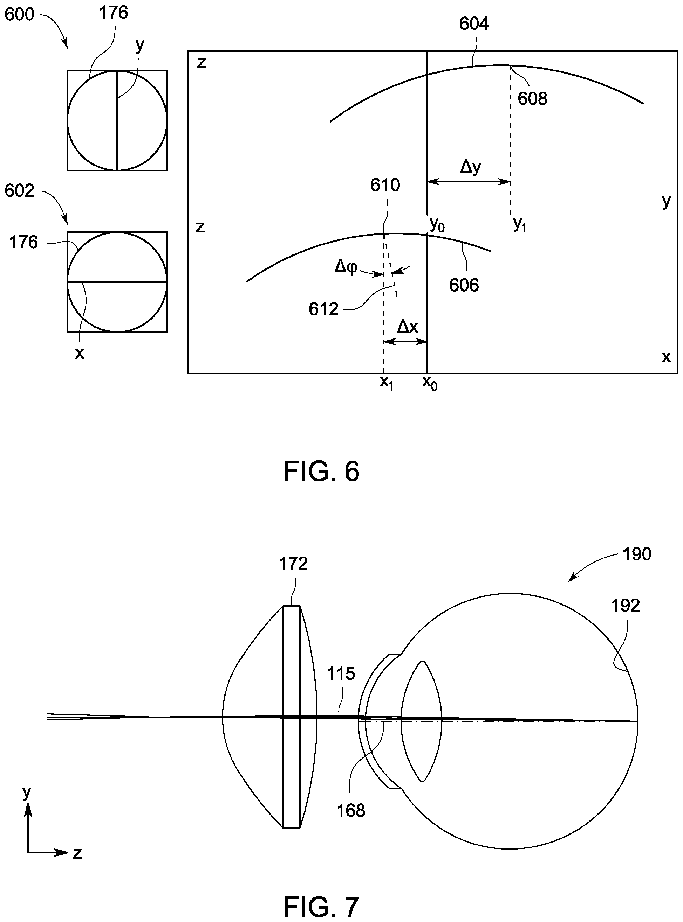

In Fig. 5, a flow scheme describing a method according to the invention in a preferred embodiment is schematically shown, and in Fig. 6, OCT images are shown. In the following, the method will be described with respect to Figs. 5 and 6. Also, reference will be made to components shown in the other Figs.

In a step 500, at least one radial scan of the surface 176 of the relay lens 172 is performed by means of the OCT imaging system, once the RVLS has been properly positioned for viewing the posterior segment (retina) of the subject eye. In Fig. 6, OCT images of two such radial scans along two imaging coordinates x, y of the OCT imaging system are shown. The upper OCT image corresponds to a radial scan 600 along the y-direction of the OCT imaging system, and the lower OCT image corresponds to a radial scan 602 along the x-direction of the OCT imaging system. Note, that a radial scan includes the apex or highest point of the surface 176 of the relay lens. Further, note that such OCT images can be presented on a display, but this is not necessary for the present invention.

In step 510, at least one curve corresponding to a shape of the surface 176 of the relay lens 172 is determined from the scan data, acquired during the radial scans 600, 602. This can include automatically segmenting the surface of the relay lens and fitting curves. In Fig. 6, a curve 604 for the scan along the y-direction and a curve 606 for the scan along the x-direction are shown. In addition, the origin of the OCT imaging system x₀, y₀, is indicated.

In step 520, a lateral offset between a center of the relay lens 172 and the origin x₀, y₀, of the OCT imaging system is determined from the curves 604, 606. This can include determining an apex 608, 610 of the curves 604, 606, i.e., there highest points in z-direction. These apices 608, 610 correspond to the (single and central) apex of the relay lens or its surface 176. The corresponding positions of the apices in x- and y-direction are indicated x₁ and y₁ in Fig. 6. The differences between the positions of the apices and the origin of the OCT imaging system are denoted Δx, Δy and correspond to or indicated the lateral offset or its respective components.

In step 530, an angular offset between the center axis of the relay lens 172 and the optical axis 168 are determined from the at least one curve. In Fig. 6, such angular offset component Δϕ is indicated by a tilted center axis 162 of the relay lens with respect to a vertical line (along z-direction) of the OCT imaging system imaging coordinates system. In case of such tilt, the curve corresponding to the surface of the relay lens is tilted in the OCT image. Note, the tilted center axis 162 is shown just for means of illustration, the corresponding curve 606, as shown, does not indicate a tilt.

In step 540, the origin of the OCT imaging system is adjusted, taking into account to the lateral offset, including its components Δx, Δy. This can include automatically changing the x and y offsets x₀, y₀ (the default origin) to the values calculated, i.e., x₁, y₁. In addition, angle offset correction can be applied. These offsets or offset values can be saved as the OCT (new or adjusted) scan origin.

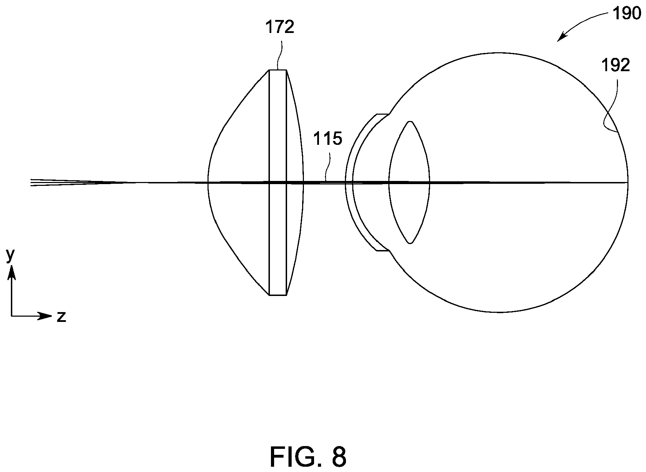

In Figs. 7 and 8, ray-trace diagrams with laterally (Fig. 7) and angularly (Fig. 8) misaligned RVLS are shown, corresponding to Figs. 3 and 4, respectively, however, the origin of the OCT imaging system being corrected as mentioned before. As can be seen, the light beam 115 reaches the optical axis 168 (and, thus, the center of the retina 192) although the RVLS is misaligned.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Some embodiments relate to a microscope comprising a system as described in connection with one or more of the Figs. 1 to 8. Alternatively, a microscope or microscopy system may be part of or connected to a system or OCT imaging system or arrangement as described in connection with one or more of the Figs. 1 to 8. Fig. 1 shows a schematic illustration of an arrangement comprising an OCT imaging system 100 configured to perform a method described herein. The arrangement comprises a microscopy system 160, an OCT imaging system 100 and a computer system or control unit 130. The microscopy system 160 and/or the OCT imaging system 100 is configured to take images and is connected to the control unit 130. The control unit 130 is configured to execute at least a part of a method described herein. The control unit 130 may be configured to execute a machine learning algorithm. The control unit 130 and microscopy system 160 and/or OCT imaging system 100 may be separate entities but can also be integrated together in one common housing. The control unit 130 may be part of a central processing system of the microscopy system 160 and/or OCT imaging system and/or the control unit 130 may be part of a subcomponent of the microscopy system 160 and/or OCT imaging system, such as a sensor, an actor, a camera or an illumination unit, etc. of the microscopy system 160 and/or OCT imaging system.

The control unit 130 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The control unit 130 may comprise any circuit or combination of circuits. In one embodiment, the control unit 130 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit Other types of circuits that may be included in the control unit 130 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The control unit 130 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The control unit 130 may also include a display device, one or more speakers, and a keyboard and/or controller, which can include a mouse, trackball, touch screen, voice-recognition device, or any other device that permits a system user to input information into and receive information from the control unit 130.

Some or all of the method steps may be executed by (or using) a hardware apparatus, like for example, a processor, a microprocessor, a programmable computer or an electronic circuit In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

### List of Reference Signs

- 100: OCT imaging system
- 102: light source
- 104: a beam splitter
- 106: reference arm
- 108, 116: lenses
- 109, 115, 117: light beams
- 110: reference mirror
- 112: sample arm
- 118: diffraction grating
- 120: detector
- 122: scan data
- 130: control system
- 132: processing means
- 140: display means
- 150: fiber optic cable
- 152: electrical cable
- 160: microscopy system
- 162: ocular
- 163: light input and output
- 164: objective
- 166: ocular
- 168: optical axis
- 170: RVLS
- 172: relay lens
- 174: reduction lens
- 176: surface of refraction lens
- 178: lateral offset
- 179: angular offset
- 190: subject eye
- 192: retina
- 194: eye lens
- 196: cornea

- 500, 510, 520, 530, 540,: method steps

- 600, 602: radial scans
- 604, 606: curves
- 608, 610: apices
- 612: center axis of relay lens

- x, y, z: imaging coordinates
- x₀, y₀: OCT imaging system origin
- x₁, y₁: positions of apices
- Δx, Δy: lateral offset components
- Δϕ: angular offset component

## Claims

1. A control system (130) for an optical coherence tomography imaging system (100) to be used with a microscopy system (160) for viewing and/or imaging a subject (190, 192), the microscopy system (160) comprising an objective (164) and a viewing lens system (170) including a relay lens (172), wherein the viewing lens system (170) is arranged at a subject's side of the objective (164), the control system (130) being configured to perform the following steps:
controlling (500) the optical coherence tomography imaging system (100) to perform at least one radial scan (600, 602) of a surface (176) of the relay lens (172),
determining (510), from data (122) of the at least one radial scan (600, 602), at least one curve (604, 606) corresponding to a shape of the surface (176) of the relay lens (172),
determining (520), from the at least one curve (604, 606), a lateral offset (178) between a center of the relay lens (172) and an origin (xo, yo) of the optical coherence tomography imaging system (100), and
adjusting (540) the origin (x₀, y₀) of the optical coherence tomography imaging system (100) taking into account to the lateral offset (178).

2. The control system (130) of claim 1, wherein the least one radial scan (600, 602) comprises at least two different radial scans, and wherein the at least one curve (604, 606) comprises at least two different curves corresponding to the shape of the surface (176) of the relay lens (172).

3. The control system (130) of claim 1 or 2, wherein determining, from the at least one curve (604, 606), the lateral offset (178) comprises: determining an apex (608, 610) of the at least one curve, determining a lateral position (x₁, y₁) of the apex, and determining the lateral offset (178) from a difference (Δx, Δy) of the lateral position of the apex and the origin of the optical coherence tomography imaging system (100).

4. The control system (130) of any one of the preceding claims, wherein determining, from the at least one curve (604, 606), the lateral offset (178) comprises: determining offset components in two different lateral directions (x, y) with respect to imaging coordinates of the optical coherence tomography imaging system (100).

5. The control system (130) of any one of the preceding claims, the control system (130) further being configured to perform the following steps:
determining (530), from the at least one curve, an angular offset (179) between a center axis (612) of the relay lens (172) and an optical axis (168) of the microscopy system (160), and
adjusting the optical coherence tomography imaging system (100) taking into account to the angular offset (179).

6. The control system (130) of any one of the preceding claims, wherein the viewing lens system (170) further includes a reduction lens (174), the reduction lens (174) being arranged between the objective (164) and the relay lens (172).

7. The control system (130) of any one of the preceding claims, wherein the subject includes or is an eye (190), in particular, a retina (192) of the eye, and wherein the viewing lens system (170) is or includes a retinal viewing lens system.

8. The control system (130) of any one of the preceding claims, wherein the viewing lens system (170) is configured as an attachable and removable accessory.

9. An arrangement comprising an optical coherence tomography imaging system (100) and a microscopy system (160) for viewing and/or imaging a subject (190, 192), the microscopy system (160) comprising an objective (164) and a viewing lens system (170) including a relay lens (172), wherein the viewing lens system (170) is arranged at the subject's side of the objective (164), further comprising the control system (130) of any one of the preceding claims.

10. A method for adjusting an optical coherence tomography imaging system (100) to be used with a microscopy system (160) for viewing and/or imaging a subject (190, 192), the microscopy system (160) comprising an objective (164) and a viewing lens system (170) including a relay lens (172), wherein the viewing lens system (170) is arranged at a subject's side of the objective (164), comprising the following steps:
performing (500) at least one radial scan of a surface (176) of the relay leans (172), by means of the optical coherence tomography imaging system (100),
determining (510), from data (122) of the at least one radial scan (600, 602), at least one curve (604, 606) corresponding to a shape of the surface (176) of the relay lens (172),
determining (520), from the at least one curve (604, 606, a lateral offset (178) between a center of the relay lens (172) and an origin (x₀, y₀) of the optical coherence tomography imaging system (100), and
adjusting (540) the origin of the optical coherence tomography imaging system (100) taking into account to the lateral offset (178).

11. The method of claim 10, wherein the viewing lens system (170) is attached to the objective (164) before performing the at least one radial scan.

12. The method of claim 10 or 11, further comprising the following steps:
determining (530), from the at least one curve (604, 606), an angular offset (179) between a center axis of the relay lens (172) and an optical axis (168) of the microscopy system (160), and
adjusting the optical coherence tomography imaging system (100) taking into account to the angular offset (179).

13. The method of any one of claims 10 to 12, wherein the viewing lens system (170) further includes a reduction lens (174), the reduction lens (174) to be arranged between the objective (164) and the relay lens (172).

14. The method of any one of claims 10 to 13, wherein the subject includes or is an eye (190), in particular, a retina (192) of the eye, and wherein the viewing lens system (170) is or includes a retinal viewing lens system.

15. A computer program with a program code for performing the method of any one of claims 10 to 14, when the computer program is run on a processor or on the control system (130) of any one of claims 1 to 9.
